# EUROPEAN PATENT APPLICATION

(11) **EP 4 464 248 A1**
(43) Date of publication of application: **20.11.2024**
(21) Application number: 22928245.4
(22) Date of filing: 21.10.2022
(51) Int. Cl.: A61B 5/293, A61B 5/31, A61B 5/294

(54) **NEURAL INTERFACE HAVING AUXILIARY STRUCTURE**

(30) Priority: 25.02.2022 CN 202210178933
(71) Applicant: Wuhan Neuracom Technology Development Co., Ltd, Wuhan, Hubei 430200 (CN)
(72) Inventor: HUANG, Li, Wuhan, Hubei 430200 (CN); HUANG, Cheng, Wuhan, Hubei 430200 (CN); JI, Junwang, Wuhan, Hubei 430200 (CN)
(74) Representative: Osterhoff, Utz
(86) International application number: PCT/CN2022/126568
(87) International publication number: WO 2023/159983

(57) **Abstract**

The present application relates to the technical field of neural interface, and in particular to a neural interface with an auxiliary structure. The neural interface includes at least one flexible electrode and at least one auxiliary structure, and the flexible electrode is assembled on one side of the auxiliary structure. The present application provides a neural interface. The auxiliary structure is rigid, and the flexible electrode is detachably assembled on the auxiliary structure, so that the flexible electrode can be easily implanted into the neural tissue with the assistance of the auxiliary structure, and the auxiliary structure is pulled out, leaving only the flexible electrode in the brain tissue. The flexible electrode can be adaptively deformed according to the expansion and contraction state of the blood vessels, reducing implantation damage.

## Description

### TECHNICAL FIELD

The present application relates to the technical field of neural interface, and in particular to a neural interface with an auxiliary structure.

### BACKGROUND

Neural interface is a communication system that does not rely on the normal output pathway composed of peripheral nerves and muscles. The neural interface bypasses the peripheral nerves and muscle tissues and provides a new pathway for exchanging information with external devices, which can stimulate nerve cells to generate action potentials through external devices, and can also record the action potentials generated by nerve cells, thereby realizing two-way communication between nerve cells and external devices. Therefore, neural interface is widely used in the research and treatment of various neurological diseases, including the human visual system.

Neural interface devices are mainly divided into implantable and non-implantable types. Compared with non-implantable neural electrodes, implantable neural electrodes have attracted the attention of domestic and foreign scholars due to their high resolution. At present, most implantable microneedle structures use electrocorticogram (EcoG), Utah electrodes, Michigan electrodes, sewing machine flexible electrodes, etc. Among implantable neural electrodes, microneedle array electrodes represented by Utah electrodes have high spatial resolution and can have the highest stability during long-term implantation, which are the only neural interface devices approved by the U.S. Food and Drug Administration (FDA) that can be used in the human body. However, when the neural interface devices in the related art are used, craniotomy is required for microneedle implantation, which is difficult, and the implantation method will also cause greater implantation damage, affecting the activity and growth of organ tissues, and being not conducive to long-term implantation. Therefore, a better solution is currently needed to solve the problems in the related art.

### SUMMARY

The main purpose of the present application is to provide a neural interface with an auxiliary structure, aiming to solve the technical problem that the current implantable neural interface microneedle array causes greater damage to brain tissue and cannot achieve long-term implantation.

In order to solve the above technical problems, according to one aspect of the present application, the present application provides the following technical solutions and provides a neural interface, including at least one flexible electrode and at least one auxiliary structure, and the flexible electrode is assembled on one side of the auxiliary structure.

In an embodiment, a ring-shaped structure is provided on the flexible electrode, and the ring-shaped structure includes a through hole and/or a recessed groove.

In an embodiment, the auxiliary structure includes an auxiliary member.

In an embodiment, the auxiliary member is accommodated in the through hole or in the recessed groove so that the flexible electrode and the auxiliary structure are tightly connected together.

In an embodiment, the auxiliary member includes a fixed portion fixed at an end of the auxiliary structure and a connection portion accommodated in the through hole.

In an embodiment, the auxiliary structure is made of biocompatible material.

In an embodiment, the auxiliary structure is an auxiliary needle accommodated in the recessed groove.

In an embodiment, the flexible electrode is provided corresponding to the auxiliary structure one-to-one.

In an embodiment, at least one slit is provided on the flexible electrode.

Compared with the related art, the present application has the following beneficial effects:
The present application provides a neural interface. The auxiliary structure is rigid, and the flexible electrode is detachably assembled on the auxiliary structure, so that the flexible electrode can be easily implanted into the neural tissue with the assistance of the auxiliary structure, and the auxiliary structure is pulled out, leaving only the flexible electrode in the brain tissue. The flexible electrode can be adaptively deformed according to the expansion and contraction state of the blood vessels, reducing implantation damage.

### BRIEF DESCRIPTION OF THE DRAWINGS

In order to illustrate the technical solutions of the present application more clearly, a brief introduction will be made below to the drawings required in the embodiments. It should be understood that the drawings only show some embodiments of the present application and should not be regarded as limiting the scope of the present application. Similar components are similarly numbered in the drawings.
FIG. 1 is a schematic decomposed structural view of a microneedle-assisted implanting neural interface according to an embodiment of the present application.
FIG. 2 is a schematic single structural view of the microneedle-assisted implanting neural interface according to an embodiment of the present application.
FIG. 3 is a schematic array structural view of the microneedle-assisted implanting neural interface according to an embodiment of the present application.
FIG. 4 is a schematic structural view of the flexible electrode according to an embodiment of the present application.
FIG. 5 is a schematic structural view of an auxiliary structure cooperated with a flexible electrode according to an embodiment of the present application.

Reference signs: 1-integrated circuit chip, 2-flexible electrode, 3-body electrode point, 4-ring-shaped structure, 5-auxiliary structure, 6-auxiliary member, 7-microneedle array.

### DETAILED DESCRIPTION OF THE EMBODIMENTS

The technical solutions in the embodiments of the present application will be clearly and completely described as followed with reference to the drawings in the embodiments of the present application. Obviously, the embodiments are only part of the embodiments of the present application, not all of the embodiments.

The components of the embodiments of the present application described and shown in the drawings can be provided and designed in various different configurations. Therefore, the following detailed description of the embodiments of the present application provided in the drawings is not intended to limit the scope of the present application, but only represents the embodiments of the present application. Based on the embodiments of the present application, all other embodiments obtained by those skilled in the art without creative efforts are within the scope of the present application.

In the following, the terms "including", "having" and their cognates that can be used in various embodiments of the present application are only intended to indicate specific features, numbers, steps, operations, elements, components or combinations of the foregoing items, and should not be understood as first excluding the existence of one or more other features, numbers, steps, operations, elements, components or combinations of the foregoing items or increasing the possibility of one or more features, numbers, steps, operations, elements, components or combinations of the foregoing items.

In addition, the terms "first", "second", "third", etc. are only used to distinguish the description and cannot be understood as indicating or implying relative importance.

Unless otherwise defined, all terms (including technical terms and scientific terms) used herein have the same meanings as those generally understood by those skilled in the art to which the various embodiments of the present application belong. The terms (such as those defined in generally used dictionaries) will be interpreted as having the same meanings as the contextual meanings in the relevant technical field and will not be interpreted as having idealized meanings or overly formal meanings unless clearly defined in the various embodiments of the present application.

### First embodiment:

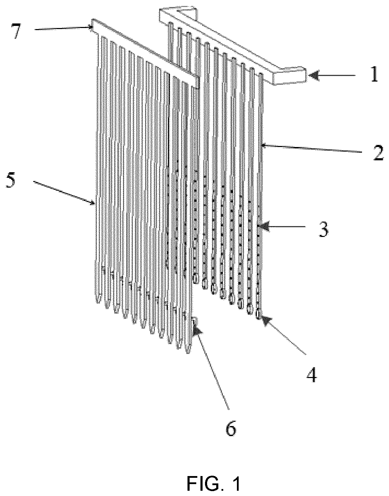

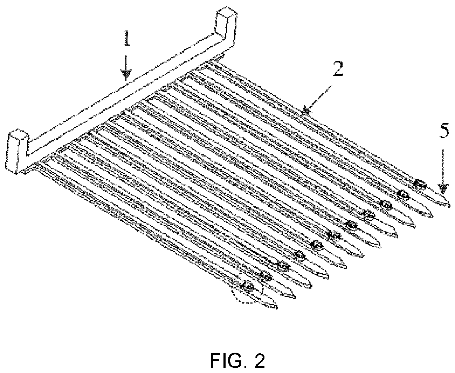

As shown in FIG. 1 and FIG. 2, the present application provides a neural interface, including an integrated circuit chip 1 and at least one flexible electrode 2. One end of the flexible electrode 2 is provided with a ring-shaped structure 4, and the ring-shaped structure includes a through hole and/or a recessed groove. The through hole has the same penetration direction as the length direction of the flexible electrode, and the flexible electrode 2 can be made of a flexible material. The present application has no particular restrictions on the material of the flexible material, as long as the material is harmless to human tissue. According to an embodiment of the present application, the material of the flexible electrode 2 is one or more of silica gel, polydimethylsiloxane (PDMS), and polyimide. The flexible electrode 2 is electrically connected to the integrated circuit chip 1, and the flexible electrode 2 includes at least one body electrode point 3, which can realize the output of signals and the input of weak electrical stimulus signals. The end of the flexible electrode 2 is provided with a ring-shaped structure 4. The present application has no special restrictions on the structure of the ring-shaped structure 4 and its connection relationship with the flexible electrode 2. The ring-shaped structure 4 can be externally connected to the flexible electrode 2, and its material can be metal, or other flexible or rigid materials. The ring-shaped structure 4 can also be integrally formed by the base material of the flexible electrode 2 and placed at the end of the flexible electrode 2.

The neural interface also includes at least one auxiliary structure 5 corresponding to the flexible electrode 2, and the flexible electrode 2 can be detachably assembled on one side of the auxiliary structure 5.

In other embodiments, the number of the auxiliary structures can also be less than the number of the flexible electrodes, as long as to assist the flexible electrode 2 implanting to the target position under the action of the auxiliary structure 5.

The body electrode point 3 is provided on the front side of the flexible electrode 2, the ring-shaped structure 4 is provided on the back side of the flexible electrode 2, and the auxiliary structure is also provided on the back side of the flexible electrode 2.

In an embodiment, the auxiliary structure 5 includes an auxiliary needle and an auxiliary member 6. The auxiliary member 6 is provided on the surface of the auxiliary needle, and the auxiliary member includes a fixed portion and a connection portion. The fixed portion is fixed to the end of the auxiliary structure, and the connection portion can be accommodated in the through hole. The auxiliary member 6 can be a hook, which can be accommodated in the through hole and can be nested with the ring-shaped structure 4 at the end of the flexible electrode 2 to form a detachable connection, so that the flexible electrode 2 and the auxiliary structure 5 are tightly connected together.

The size of the auxiliary member 6 in the present application is very small, and the damage to the organ tissue when inserted with the auxiliary structure 5 can be basically ignored. When in use, the ring-shaped structure 4 at the end of the flexible electrode 2 is hung on the auxiliary member 6 of the auxiliary structure 5, and is inserted into the brain tissue together. The auxiliary structure 5 is pulled out, the auxiliary member 6 is separated from the ring-shaped structure 4 of the flexible electrode 2, the flexible electrode 2 remains in the brain tissue, and the auxiliary structure 5 is pulled out of the body, which will not cause long-term implanting damage.

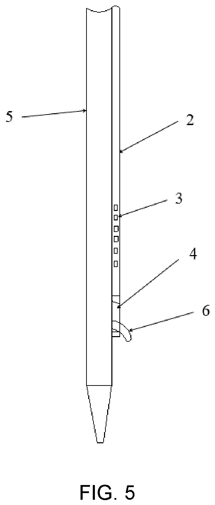

As shown in FIG. 5, in an embodiment of the present application, the inner ring surface of the top of the ring-shaped structure 4 is an inclined structure, and is inclined towards the side attached to the auxiliary structure 5. The inner and outer sides of the hook have a certain curvature, which is convenient for unhooking and prevents the flexible electrode 2 from being brought out when the auxiliary structure 5 is pulled out.

In other embodiments, the auxiliary member can be a protrusion accommodated in the recessed groove, and the protrusion forms a detachable connection with the recessed groove. In this embodiment, a blocking portion is provided at the lower end of the recessed groove to prevent the protrusion from sliding out of the recessed groove.

The auxiliary member 6 is formed of silicon dioxide or silicon nitride. The hook can be formed in the following manner: after the auxiliary structure body is made, a recessed groove is etched at a set position of the auxiliary structure body, then a pattern of the hook is defined by photoresist; starting from the recessed groove, silicon dioxide or silicon nitride is grown along the predefined pattern to form a hook, and the remaining photoresist is removed.

In an embodiment, the auxiliary structure includes an auxiliary needle accommodated in the recessed groove. The length of the recessed groove is relatively longer, and the length of the recessed groove is matched with the length of the auxiliary needle.

In an embodiment, the flexible electrode can use a flexible material as a substrate to ensure that the flexible electrode can be deformed under the action of an external force. The flexible material includes PDMS to ensure that the electrode contacts the neural tissue in a softer state.

In an embodiment, at least one slit is provided on the flexible electrode. When at least two slits are provided on the flexible electrode, the slits are provided at intervals along the length direction of the flexible electrode. The two adjacent slits are staggered, and the slits are perpendicular to or inclined to the length direction of the flexible electrode, so that the flexible electrode 2 can be deformed.

Specifically, the flexible electrode 2 can use a rigid material as the substrate, and the body electrode specifically includes a base plate and a plurality of slits distributed on the base plate. The plurality of slits are provided at intervals along the length direction of the body electrode (hereinafter described as the first direction for ease of description), two adjacent slits are staggered, and the slits are perpendicular to or inclined to the first direction. The slit can be perpendicular to the length direction of the body electrode (i.e., the first direction) or inclined to the length direction of the body electrode. Arranging the slit on the base plate can reduce the rigidity of the entire body electrode to a certain extent, but the size of the slit needs to be controlled within a certain range and cannot be too small, otherwise it will cause lack of flexibility.

Here, the shape of the slit is not specifically limited, which can be a long strip, a wave shape, a sawtooth shape, etc.

The auxiliary structure 5 of the neural interface of this embodiment is rigid, and the flexible electrode 2 is detachably assembled on the auxiliary structure 5, so that the flexible electrode 2 can be easily implanted into the neural tissue with the assistance of the auxiliary structure 5. The auxiliary structure is pulled out, leaving only the flexible electrode in the brain tissue. The flexible electrode can be adaptively deformed according to the expansion and contraction state of the blood vessels to reduce implantation damage.

### Second embodiment:

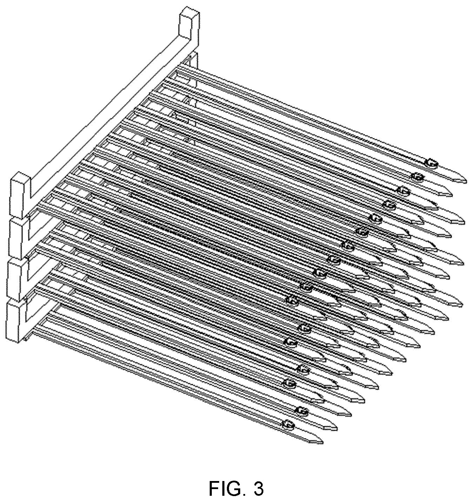

As shown in FIG. 1 and FIG. 2, in an embodiment of the present application, a plurality of auxiliary structures 5 are provided to form a microneedle array 7, and a plurality of flexible electrodes 2 are provided, which are bonded to the integrated circuit chip 1 to form an electrode array. The spacing between the auxiliary structures 5 of the microneedle array 7 is the same as the spacing between the flexible electrodes 2 bonded to the integrated circuit chip 1. When the microneedle array 7 is attached to the flexible electrode array, the auxiliary structure 5 and the flexible electrode 2 can correspond one to one, realizing the precise matching of the ring-shaped structure 4 and the auxiliary member 6, and the flexible electrode 2 is in a taut state. The microneedle array 7 and the flexible electrode array are attached and combined to form a microneedle electrode assembly. As shown in FIG. 3, a plurality of microneedle electrode assemblies can be combined to form a microneedle electrode array, which can form regional coverage when implanting a large brain tissue, thereby improving spatial resolution and signal accuracy.

### Third embodiment:

In an embodiment of the present application, the microneedle array includes at least two auxiliary structures 5 of unequal lengths, and the positions of the corresponding auxiliary members 6 may be on the same plane or not.

In an embodiment of the present application, the position of the auxiliary member 6 on the auxiliary structure 5 is adjustable. The present application has no particular restrictions on the adjustment method, which may be nested sliding adjustment or installation positioning. According to an embodiment of the present application, a plurality of mounting holes are provided on the auxiliary structure 5, which are provided in the length direction of the auxiliary structure, and the auxiliary member 6 can be fixed in the mounting holes by snapping, so that the snapping position of the auxiliary member 6 can be set according to the length of the flexible electrode 2 to be implanted.

In an embodiment of the present application, the flexible electrode 2 can be cut, and can be cut according to the length of the auxiliary structure 5 and the position of the auxiliary member 6 to obtain flexible electrodes 2 of different lengths. One side of the flexible electrode 2 without the ring-shaped structure 4 is cut, and a protective film is provided at the fracture of the cut flexible electrode 2, or the flexible electrode 2 is re-bonded with the integrated circuit chip 1.

### Fourth embodiment:

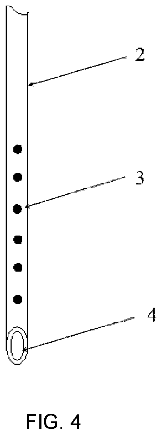

As shown in FIG. 4 and FIG. 5, the flexible electrode 2 includes at least one body electrode point 3 independent of each other and can work independently under the control of the integrated circuit chip 1. A plurality of body electrode points 3 are provided on the flexible electrode 2, so that the signal reading and stimulation can be carried out simultaneously. In an embodiment, the body electrode points 3 on the flexible electrode 2 can be distributed in the same column or in different columns, which can be determined according to the width of the flexible electrode 2 and the actual situation.

In an embodiment, the neural interface further includes an in vitro device, including a collecting unit and a processing unit. The collecting unit is configured to obtain external sensory signals, and the processing unit is configured to convert sensory signals into stimulus signals for reproducing sensory signals.

In an embodiment, the in vitro device also includes a first wireless coil, and a second wireless coil is provided on the side of the integrated circuit chip. The first wireless coil is configured to send stimulus signals, and the second wireless coil is configured to receive stimulus signals.

### Fifth embodiment:

According to an embodiment of the present application, a method for implanting a neural interface is also provided, and the specific technical scheme is as follows:
(1) selecting auxiliary structures 5 of different lengths or adjusting the position of auxiliary members 6 on the auxiliary structure 5 according to the implantation depth of the flexible electrode 2;
(2) sleeving the ring-shaped structure 4 of the flexible electrode 2 on the auxiliary member 6 of the auxiliary structure 5 to make the auxiliary structure 5 attached to the flexible electrode 2 and the integrated circuit chip 1, and adjusting the flexible electrode 2 to a taut state;
(3) inserting a combined structure of the auxiliary structure 5 and the flexible electrode 2 into the brain tissue; and
(4) adjusting the flexible electrode 2 to a relaxed state, pulling out the auxiliary structure 5, and realizing the decoupling process.

In an embodiment of the present application, the inner ring surface of the top of the ring-shaped structure described in step (2) is inclined towards the side attached to the auxiliary structure 5, so as to facilitate the decoupling process of step (4).

The method of implanting the flexible electrode 2 with assistance adopted in the present application has less implant damage and can increase the stability of long-term implanting. The flexible electrode 2 includes the plurality of body electrodes, which can adapt to the structural deformation of the brain tissue, avoid the strange feeling caused by friction with tissues and organs, and can also realize multi-electrode recording, thus improving spatial resolution and signal accuracy.

The above-mentioned embodiments only express several implementation methods of the present application, and the description is relatively specific and detailed, but it cannot be understood as a limitation on the scope of the present application. It should be pointed out that for those skilled in the art, several deformations and improvements can be made without departing from the concept of the present application, which all belong to the scope of the present application. Therefore, the scope of the present application shall be based on the attached claims.

## Claims

1. A neural interface, **characterized by** comprising: at least one flexible electrode and at least one auxiliary structure, wherein the flexible electrode is assembled on one side of the auxiliary structure.

2. The neural interface according to claim 1, wherein a ring-shaped structure is provided on the flexible electrode, and the ring-shaped structure comprises a through hole and/or a recessed groove.

3. The neural interface according to claim 2, wherein the auxiliary structure comprises an auxiliary member.

4. The neural interface according to claim 3, wherein the auxiliary member is accommodated in the through hole or in the recessed groove so that the flexible electrode and the auxiliary structure are tightly connected together.

5. The neural interface according to claim 3, wherein the auxiliary member comprises a fixed portion fixed at an end of the auxiliary structure and a connection portion accommodated in the through hole.

6. The neural interface according to claim 3, wherein a material of the auxiliary member comprises silicon dioxide or silicon nitride.

7. The neural interface according to claim 2, wherein the auxiliary structure is an auxiliary needle accommodated in the recessed groove.

8. The neural interface according to any one of claims 1 to 7, wherein the auxiliary structure is made of biocompatible material.

9. The neural interface according to any one of claims 1 to 7, wherein the flexible electrode is provided corresponding to the auxiliary structure one-to-one.

10. The neural interface according to any one of claims 1 to 7, wherein at least one slit is provided on the flexible electrode.
